# EUROPÄISCHE PATENTSCHRIFT

(11) **EP 4 231 894 B1**
(45) Veröffentlichungstag und Bekanntmachung des Hinweises auf die Patenterteilung: **16.10.2024**
(21) Anmeldenummer: 21800996.7
(22) Anmeldetag: 21.10.2021
(51) Int. Cl.: A61B 3/06, A61B 3/00

(54) **COMPUTERIMPLEMENTIERTES VERFAHREN ZUR OPTOMETRISCHEN FARBAUSTESTUNG**
COMPUTER-IMPLEMENTED METHOD FOR OPTOMETRIC COLOR TESTING
PROCÉDÉ MIS EN OEUVRE PAR ORDINATEUR POUR UN TEST DE COULEUR OPTOMÉTRIQUE

(30) Priorität: 21.10.2020 AT 509052020
(43) Veröffentlichungstag der Anmeldung: 30.08.2023
(73) Patentinhaber: BISCHEL, Roland, 2514 Traiskirchen (AT)
(72) Erfinder: BISCHEL, Roland, 2514 Traiskirchen (AT)
(74) Vertreter: SONN Patentanwälte GmbH & Co KG
(86) Internationale Anmeldenummer: PCT/AT2021/060390
(87) Internationale Veröffentlichungsnummer: WO 2022/082247

(56) Entgegenhaltungen:
- WO-A1-2012/120256
- WO-A1-2019/099952
- JP-A- 2005 346 273
- KR-B1- 101 768 071
- US-B1- 9 826 898

## Beschreibung

Die Erfindung betrifft ein computerimplementiertes Verfahren zur optometrischen Farbaustestung. Das Verfahren kann beispielsweise als motorischer Test einer individuellen optimalen Lichtumgebung ausgebildet sein.

Im Stand der Technik bekannte Verfahren zur Untersuchung des Gesichtsfelds befassen sich im Allgemeinen nicht mit einer optometrischen Farbaustestung und liegen daher auf einem anderen technischen Gebiet.

Die US 10,209,773 B2 beschreibt verschiedene Verfahren zum Testen der individuellen Sehleistung einer Person. Bei dem als Runde 2 eines Spiels beschriebenen Verfahren werden auf einem Bildschirm Ballons unterschiedlicher Farbe auf unterschiedlichen Hintergründen für kurze Zeit angezeigt und der Benutzer muss die Ballons treffen, wobei die Reaktionsgeschwindigkeit und die Treffergenauigkeit erfasst werden. Diese Parameter werden anschließend zur Ermittlung einer allgemeinen Sehschärfe verwendet. Es ist kein Vergleich von verschiedenen Farbkombinationen vorgesehen.

In der US 2017/0169716 A1 ist ein Verfahren und System gezeigt, bei dem motorische Reaktionen für Tests der visuellen Wahrnehmung ausgewertet werden. Im Einzelnen werden auf einem Bildschirm Symbole an verschiedenen, unvorhersehbaren Positionen angezeigt und der Benutzer muss die wahrgenommene Position des angezeigten Symbols eingeben. Die Positionen sind auf drei diskrete Optionen beschränkt: Links, Mitte, Rechts. Ein Vergleich von verschiedenen Farbkonfigurationen unter Berücksichtigung einer Korrelation zwischen der Farbe der angezeigten Symbole und der Trefferquote der Antworten ist nicht vorgesehen.

Die US 2018/0310820 A1 offenbart einen Test der visuellen Wahrnehmung einer Person, im Zuge dessen Symbole unterschiedlicher Farben angezeigt werden können. Zur Ermittlung eines individuellen optischen Parameters wird mit einer Kamera die Haltung und Blickrichtung der Testperson erfasst und ausgewertet. Eine Eingabe der Positionen der angezeigten Symbole ist nicht vorgesehen.

Die WO 2012/120256 A1 offenbart ein Verfahren zur Ermittlung einer optimalen Farbkombination einer Hintergrundfarbe für ein Bild. Dazu wird eine erste Farbkombination von Bild und Hintergrundfarbe dargestellt und gleichzeitig wird eine Stoppuhr gestartet. Die Stoppuhr wird angehalten, sobald der Nutzer das Bild wahrnimmt und die Zeit wird als erste Reaktionszeit gespeichert. Dieser Vorgang wird für eine weitere Farbkombination wiederholt. Als optimal wird jene Farbkombination ermittelt, bei welcher die kürzeste Reaktionszeit gemessen wurde.

Die US 9826898 B1 offenbart unter anderem ein Verfahren zur Ermittlung von Farbsehschwächen. Dafür wird ein Bildschirm mit Touch-Funktion eingesetzt, auf dem eine Hintergrundfarbe sowie ein Farbfleck oder mehrere Farbflecken abgebildet sind. Die Erfindung soll insbesondere zur Erkennung der Farbsehschwächen Protanomalie, Deuteranomalie und Tritanomalie und deren Schwere eingesetzt werden können. Dafür werden die Hintergrundfarbe und der Farbfleck so ausgewählt, dass sie schwer unterscheidbar für Menschen mit einer entsprechenden Farbsehschwäche sind. Die Flecken sollen durch Anklicken identifiziert werden. Wählt ein Nutzer einen entsprechenden Fleck nicht aus, kann darauf geschlossen werden, dass er die entsprechende Farbsehschwäche hat. Durch unterschiedliche "Schwierigkeitsstufen" bei der Erkennung der Flecken für jede Form der Sehschwäche, kann nicht nur ermittelt werden welche Sehschwäche vorliegt, sondern auch, wie stark sie ausgeprägt ist.

Das in der JP 2005 346273 A gezeigte Verfahren macht es sich zu Nutze, dass es für Menschen mit Farbsehschwäche möglich ist, Farben zu unterscheiden, indem visuell gelehrt wird, wie sehr sich die Farben in einem Graustufenbild oder einem Abstufungsbild ähnlicher Farben unterscheiden. Der Nutzer mit einer Farbsehschwäche kann ein Foto von seiner Umgebung machen. Dieses wird anschließend in ein Graustufenbild umgewandelt. Das Graustufenbild und das Originalfoto werden abwechselnd angezeigt, jeweils für einen sehr kurzen Zeitraum, sodass der Nutzer anhand des Graustufenbilds ausmachen kann, was auf dem Originalfoto tatsächlich zu sehen ist.

Die KR 10-1768071 B1 zeigt ein Verfahren zur Korrektur der Farbschwäche eines Achromats durch Messung des Grades der Farbschwäche des Achromats.

Ein weiteres Verfahren, das allerdings keine motorische Komponente vorsieht, ist der Lantern Test nach Beyne.

Bei ca. 10-12 % der Bevölkerung liegt eine Überempfindlichkeit gegen einzelne Lichtfrequenzen vor - das Irlen Syndrom. Die Reizverarbeitung im Gehirn ist dann anstrengend und ermüdend; manche Betroffene leiden sogar an Lese- oder Schreibproblemen. Abhilfe kann eine individuell angepasste optometrische Farbbrille/Sonnenbrille schaffen. Auch die Anpassung der Farbeinstellungen am Bildschirm (z.B. am Arbeitsplatz oder Smartphone) kann Erleichterung schaffen. Dazu ist die Austestung des optometrischen Farbtyps nötig.

Die Praxis zeigt, dass eine geeignete Korrektur mittels Farbfiltergläsern für die Betroffenen nicht selten einen Wendepunkt darstellt. Konzentrationsfähigkeit, Motivation und Leistungsfähigkeit steigen. Menschen finden den Zugang zum Lesen. Lernprobleme und Legasthenie entschärfen sich usw. Studien zeigen, dass 46 % aller Menschen, die eine diagnostizierte Lese- oder Lernschwäche, Legasthenie oder ADHS haben, von einer Brille mit Farbfiltergläsern profitieren können.

Eine Aufgabe des offenbarten Verfahrens ist, mit weitverbreiteten Mitteln (z.B. ein Smartphone oder Tablett-PC) einen individuell optimalen Farbton für eine Lichtumgebung z.B. im Auto-Innenraum oder am Computerbildschirm zu ermitteln.

Das erfindungsgemäße Verfahren der eingangs angeführten Art umfasst folgende Schritte: Messen einer Abweichung einer zeitverzögerten Positionsschätzung eines Nutzers für jede von mindestens zwei verschiedenen Farbkonfigurationen; und Ermitteln einer für den Nutzer empfohlenen Farbkonfiguration aus den gemessenen Abweichungen. Aus der empfohlenen Farbkonfiguration lässt sich ein Farbton für eine Lichtumgebung ablesen, indem z.B. für ein Umgebungslicht eine Tönung in der empfohlenen Hintergrundfarbe gewählt wird. Die zeitverzögerte Positionsschätzung kann beispielsweise durch eine Benutzereingabe einer Position erfasst werden. Dass die Schätzung zeitverzögert ist, bedeutet, dass der Benutzer die Position aus seiner Erinnerung heraus eingibt. Insofern könnte die zeitverzögerte Positionsschätzung aus Sicht des Nutzers auch als Positionserinnerung bezeichnet werden, die gemessen wird. Geschätzt wird dabei eine Position, über die der Nutzer zuvor - in einem Zeitabstand - informiert oder die ihm mitgeteilt wurde, sodass er sich später daran erinnern kann. Die Abweichung bezeichnet dementsprechend ein Maß für einen Unterschied zwischen der mitgeteilten Position und der geschätzten Position. Das gegenständliche Verfahren basiert auf der Erkenntnis, dass sich die Zeitverzögerung auf die Positionsschätzung auswirkt und außerdem, dass diese Auswirkung für verschiedene Farbkonfigurationen verschieden ist. Es stellt sich heraus, dass diese Auswirkung und deren Abhängigkeit von der Farbkonfiguration charakteristisch für die Person des Nutzers ist. Daher kann mit dem offenbarten Verfahren eine individuell, für den jeweiligen Nutzer angepasste Farbkonfiguration ermittelt und empfohlen werden. Mit diesem Verfahren kann auf Basis der Testergebnisse eine Reihung der getesteten Farbkombinationen vorgenommen werden. Im Zuge des Verfahrens können insbesondere die gemessenen Zeiten und Genauigkeiten mit den angezeigten Farben verknüpft aufgezeichnet werden.

Für eine genauere Aussage können im Rahmen des offenbarten Verfahrens auch mindestens drei, mindestens vier oder mindestens fünf verschiedene Farbkonfigurationen verwendet werden, um jeweils eine Abweichung einer zeitverzögerten Positionsschätzung eines Nutzers zu messen. Es könnten auch beispielsweise mindestens zehn verschiedene Farbkonfiguration verwendet werden. Alternativ oder zusätzlich zu einer vordefinierten Reihe von Farbkonfiguration könnten zusätzliche verschiedene Farbkonfiguration zufällig generiert werden. Die Anzahl der verwendeten Farbkonfiguration könnte auch abhängig von den Ergebnissen der ersten beiden Messungen dynamisch vergrößert werden, z.B. bis eine vordefinierte Signifikanz einer statistischen Auswertung für die Ermittlung der empfohlenen Farbkonfiguration erreicht wird. Beispielsweise könnten bei geringen Unterschieden zwischen zwei bestimmten Farbkonfiguration zusätzliche Messungen durchgeführt werden, bevor eine Empfehlung zwischen diesen beiden Farbkonfiguration abgegeben wird. Andererseits könnte bei großen Unterschieden der gemessenen Abweichungen für die betrachteten Farbkonfiguration bereits mit wenigen Messungen eine empfohlene Farbkonfiguration ermittelt werden.

Dabei kann das Ermitteln der für den Nutzer empfohlenen Farbkonfiguration folgende Schritte umfassen: Vergleichen der gemessenen Abweichungen; und Ermitteln mindestens einer Farbkonfiguration, der eine geringere Abweichung zugeordnet ist als mindestens einer anderen Farbkonfiguration, als empfohlene Farbkonfiguration. Als Abweichung kann beispielsweise der geometrische Abstand zwischen der mitgeteilten Position und der Positionsschätzung verwendet werden. Dadurch sind die Abweichungen als Skalar unmittelbar vergleichbar. Indem eine Farbkonfiguration mit einer relativ geringeren Abweichung der zeitverzögerten Positionsschätzung eher empfohlen wird, werden solche Farbkonfigurationen bevorzugt empfohlen, bei denen der Nutzer eine genaue Positionserinnerung hat, was eine genaue Positionswahrnehmung voraussetzt. Die Verwendung einer solchen Farbkonfiguration hilft dem Nutzer dabei, Positionsinformationen, die in dieser Farbkonfiguration angezeigt werden, einfacher und genauer wahrzunehmen und ist daher empfehlenswert. Da Positionsinformationen in vielen Anwendungsbereichen bedeutungstragend sind (z.B. Schriften, Diagramme, Tabellen, Zeichnungen, Karten, etc.), stellt die Kenntnis einer individuell besonders geeigneten Farbkonfiguration zu deren Anzeige einen wesentlichen Vorteil für den Nutzer dar.

Im Rahmen des offenbarten Verfahrens kann das Messen der Abweichungen der zeitverzögerten Positionsschätzung je Farbkonfiguration beispielsweise folgende Schritte umfassen: Anzeigen eines Positionshinweises in der jeweiligen Farbkonfiguration an einer Anzeigeposition auf einer elektronisch gesteuerten Anzeige; Ausblenden des Positionshinweises; Erfassen einer Positionseingabe einer Eingabeposition mit einem Positionseingabegerät nach dem Ausblenden des Positionshinweises; und Ermitteln einer Abweichung zwischen der Eingabeposition und der Anzeigeposition als Abweichung der zeitverzögerten Positionsschätzung. Die Verwendung einer elektronisch gesteuerten Anzeige ermöglicht das Ein- und Ausblenden von Informationen, wie dem gegenständlichen Positionshinweis. Das Ausblenden des Positionshinweises kann beispielsweise nach einer vordefinierten Anzeigedauer erfolgen. Indem die Anzeigedauer vorab definiert wird, kann die Vergleichbarkeit der Farbkonfigurationen verbessert und die Reproduzierbarkeit der Messung erhöht werden. Aufgrund der elektronischen Steuerung kann sichergestellt werden, dass die Positionseingabe erst nach dem Ausblenden des Positionshinweises erfolgt und nicht bei noch angezeigten Positionshinweis. Dadurch kann vermieden werden, dass anstelle der Positionserinnerung nur die Eingabegenauigkeit gemessen wird. Die Anzeige und das Positionseingabegerät können beispielsweise durch einen Touch-Bildschirm gebildet sein. Die vordefinierte Anzeigedauer kann zwischen 0,1 und 20 Sekunden, insbesondere zwischen einer und 5 Sekunden, beispielsweise bei 2 Sekunden liegen. Die Abweichung kann beispielsweise als geometrische Distanz zwischen der Eingabeposition und der Anzeigeposition ermittelt werden.

Gemäß einer optionalen Ausführungsvariante kann das Erfassen einer Positionseingabe folgende Schritte umfassen: Anzeigen einer Eingabemarkierung an einer von der Anzeigeposition verschiedenen Markierungsposition auf der elektronisch gesteuerten Anzeige; und Erfassen einer Streckeneingabe einer Verbindungsstrecke von der Markierungsposition bis zu der zu erfassenden Eingabeposition mit dem Positionseingabegerät. Beispielsweise kann der Nutzer einen Positionsangaben durchführen, in dem eine Eingabemarkierung von der Markierungsposition zur beabsichtigten Eingabeposition "gezogen" wird. Bei dieser Variante kann vermieden werden, dass der Nutzer bereits bei der Anzeige des Positionshinweises eine Positionsangabe vorbereitet (beispielsweise einen Finger an der Position des Positionshinweises über einen Touch-Bildschirm bereithält). Vielmehr wird erreicht, dass eine motorische Aktion innerhalb der relevanten Dimensionen (d. h. in der Ebene des Positionshinweises und der Eingabeposition) notwendig ist und anhand der Positionserinnerung gesteuert werden muss. Bei dieser Variante kann das Ausblenden des Positionshinweises bei einer Annäherung der Streckeneingabe an die Anzeigeposition erfolgen und optional unabhängig von einer etwaigen vordefinierten Anzeigedauer.

Im Rahmen des offenbarten Verfahrens kann vorgesehen sein, dass das Verfahren umfasst: Speichern der erfassten Eingabeposition oder einer ermittelten Abweichung mit einer Zuordnung zu der jeweiligen Farbkonfiguration. Nach der Durchführung des Verfahrens für verschiedene Farbkonfigurationen können diese anschließend anhand der zugeordneten gespeicherten Daten verglichen werden. Weiters können zu verschiedenen Zeitpunkten gemessenen Eingabepositionen bzw. Abweichungen mit derselben Farbkonfiguration verknüpft oder diese zugeordnet werden, sodass eine zeitliche Entwicklung und/oder Statistik über verschiedene Messungen mit Bezug zur selben Farbkonfiguration ermittelt werden kann.

Optional kann vorgesehen sein, dass das Erfassen einer Positionseingabe nach einem vordefinierten Zeitabstand nach dem Ausblenden des Positionshinweises beginnt. Frühere Positionseingabeversuche, d. h. die noch während des angezeigten Positionshinweises oder innerhalb des vordefinierten Zeitabstand erfolgen, werden abgelehnt oder ignoriert oder führen dazu, dass die Messung abgebrochen wird, allenfalls mit einem darauf folgenden Äxten der Farbkonfiguration für eine neue Messung.

Das offenbarte Verfahren kann umfassen: Ausgeben eines Bereitschaftssignals nach dem Ausblenden des Positionshinweises. Das Bereitschaftssignal signalisiert dem Nutzer, dass eine Positionsangabe sofort oder nach einem vordefinierten Zeitabstand akzeptiert wird. Das Bereitschaftssignal können optisches, akustisches oder optisches Signal oder eine Kombination von mehreren solcher Signale sein.

Optional umfasst das offenbarte Verfahren auch: Erfassen einer Eingabeverzögerung zwischen dem Ausgeben des Bereitschaftssignals und der Positionseingabe. Die Eingabeverzögerung bezeichnet den Zeitabstand zwischen dem Zeitpunkt der Positionseingabe, genauer deren Registrierung, und dem Zeitpunkt des Bereitschaftssignals oder allgemein dem Zeitpunkt des Ausblenden Positionshinweises. Die Eingabeverzögerung kann für den Vergleich der Farbkonfiguration und für die Ermittlung einer Empfehlung berücksichtigt werden.

Im Rahmen des offenbarten Verfahrens kann vorgesehen sein, dass das Verfahren umfasst: Speichern der erfassten Eingabeverzögerung mit einer Zuordnung zu der jeweiligen Farbkonfiguration. Nach der Durchführung des Verfahrens für verschiedene Farbkonfigurationen können diese anschließend anhand der zugeordneten gespeicherten Daten verglichen werden. Weiters können zu verschiedenen Zeitpunkten gemessenen Eingabeverzögerungen mit derselben Farbkonfiguration verknüpft oder diese zugeordnet werden, sodass eine zeitliche Entwicklung und/oder Statistik über verschiedene Messungen mit Bezug zur selben Farbkonfiguration ermittelt werden kann. Bei dem Vergleich von Farbkonfiguration können die zugeordneten Eingabeverzögerungen, optional zusammen mit den zugeordneten Eingabepositionen bzw. Abweichungen berücksichtigt werden, um eine empfohlene Farbkonfiguration zu ermitteln.

Optional kann im Rahmen des offenbarten Verfahrens vorgesehen sein, dass die Anzeigeposition bei jeder Messung zufällig innerhalb zumindest eines Teils des Anzeigebereichs der elektronisch gesteuerten Anzeige festgelegt wird. Eine zufällige Festlegung der Anzeigeposition ermöglicht eine Variation zwischen aufeinanderfolgenden und wiederholten Messungen. Es wird dadurch für den Benutzer unvorhersehbar, was die nächste einzige Position sein wird. Zudem wird eine Korrelation zwischen der Antriebposition und der Farbkonfiguration vermieden, die die Messung verfälschen könnte.

Das offenbarte Verfahren, optional vorsehen, dass die Farbkonfiguration aus einer vordefinierten Liste von Farbkonfigurationen durchlaufen werden, wobei die Reihenfolge der Farbkonfigurationen vor jedem Durchlauf zufällig festgelegt wird. Eine zufällige Variation der Abfolge der Farbkonfiguration ermöglicht, dass Auswirkungen auf die Messung für eine Farbkonfiguration durch eine vorangegangene Farbkonfiguration vermieden werden oder zumindest durch eine statistische Auswertung wiederholte Messungen in verschiedenen Reihenfolgen unterdrückt werden können.

Die vordefinierte Anzeigedauer kann kürzer als 1 Sekunde sein, insbesondere kürzer als 500 Millisekunden, vorzugsweise kürzer als 100 Millisekunden ist. Durch eine kurze Anzeigedauer kann verhindert werden, dass der Nutzer eine Positionsangabe genau vorbereiten kann, während der Positionshinweises noch angezeigt wird.

Weiters betrifft die vorliegende Erfindung auch eine Vorrichtung zur Datenverarbeitung, z.B. Handy, Tablet, Laptop, PC oder AR-System, umfassend Mittel zur Ausführung der Schritte des Verfahrens nach einer der oben beschriebenen Varianten.

Außerdem betrifft die vorliegende Erfindung ein Computerprogrammprodukt, umfassend Befehle, die bei der Ausführung des Programms durch einen Computer diesen veranlassen, die Schritte des Verfahrens nach einer der oben beschriebenen Varianten auszuführen.

Schließlich betrifft die vorliegende Erfindung auch ein computerlesbares Speichermedium, umfassend Befehle, die bei der Ausführung durch einen Computer diesen veranlassen, die Schritte des Verfahrens nach einer der oben beschriebenen Varianten auszuführen.

Bei dem offenbarten Verfahren kann beispielsweise ein lokalisiertes Symbol (z.B. ein Punkt oder Kreis oder Quadrat) in verschiedenen Kombinationen einer Vordergrund- und Hintergrundfarbe angezeigt werden, eine jeweils zeitversetzte Eingabe der wahrgenommenen Position des Symbols erfasst werden und aus der Genauigkeit der eingegebenen Position im Vergleich zur angezeigten Position sowie aus der Geschwindigkeit der Eingabe eine individuelle Präferenz hinsichtlich Farbhelligkeit und Farbton ermittelt werden. Die Farbkonfiguration entspricht in diesem Fall einer Kombination einer Vordergrundfarbe mit einer Hintergrundfarbe, wobei das Symbol in der Vordergrundfarbe dargestellt wird und der Hintergrund des Symbols, d. h. ein Bildbereich in der Umgebung des Symbols in der Hintergrundfarbe eingestellt wird. Im Allgemeinen sind auch Farbkonfigurationen mit mehr als zwei Farben denkbar. In dem oben genannten Beispiel könnte beispielsweise ein blauer Kreis auf einem roten Hintergrund dargestellt werden. Die Farbkonfiguration wäre dann "blau, rot", wobei die erstgenannte Farbe die Vordergrundfarbe und die zweitgenannte Farbe die Hintergrundfarbe ist. Eine davon verschiedene Farbkonfiguration wäre beispielsweise "blau, grün". Ebenfalls eine verschiedene Farbkonfiguration wäre "rot, blau", d. h. wobei Vordergrund- und Hintergrundfarbe vertauscht sind. Wieder andere, verschiedene Farbkonfigurationen wären "blau, schwarz" und "blau, weiß" oder auch "weiß, rot". Zusätzlich zu der Verwendung von zumindest zwei verschiedenen Farbkonfigurationen wäre auch denkbar, dieselbe Farbkonfiguration mit verschiedenen Symbolen für ergänzende Messungen zu verwenden.

Die Erfindung wird nachfolgend anhand von besonders bevorzugten Ausführungsbeispielen, auf die sie jedoch nicht beschränkt sein soll, und unter Bezugnahme auf die Zeichnungen noch weiter erläutert. Dabei zeigen im Einzelnen:
Fig. 1 schematisch einen Tablet-PC bei einem ersten Schritt eines ersten Ausführungsbeispiels des offenbarten Verfahrens;
Fig. 2 schematisch den Tablet-PC gemäß Fig. 1 bei einem zweiten Schritt des ersten Ausführungsbeispiels des offenbarten Verfahrens mit einer Positionseingabe;
Fig. 3 schematisch eine Eingabeposition nach dem Schritt gemäß Fig. 2;
Fig. 4 schematisch eine Sammlung von vier Eingabepositionen nach mehreren Wiederholungen der Schritte gemäß Figuren 1 und 2 für vier verschiedene Farbkonfiguration;
Fig. 5 schematisch und vereinfacht eine Tabelle von gemessenen Abweichungen und einer daraus errechneten Treffergenauigkeit auf Basis der Eingabepositionen gemäß Fig. 4;
Fig. 6 schematisch ein Balkendiagramm der Treffergenauigkeit gemäß Fig. 5;
Fig. 7 schematisch einen Tablet-PC bei einem ersten Schritt eines zweiten Ausführungsbeispiels des offenbarten Verfahrens;
Fig. 8 schematisch den Tablet-PC gemäß Fig. 7 einem zweiten Schritt des zweiten Ausführungsbeispiels des offenbarten Verfahrens während einer Positionsangabe;
Fig. 9 schematisch eine Eingabeposition nach dem Schritt gemäß Fig. 8;
Fig. 10 schematisch eine Sammlung von vier Eingabepositionen nach mehreren Wiederholungen der Schritte gemäß Figuren 7 und 8 für vier verschiedene Farbkonfiguration;
Fig. 11 schematisch und vereinfacht eine Tabelle von gemessenen Abweichungen und einer daraus errechneten Treffergenauigkeit auf Basis der Eingabepositionen gemäß Fig. 10; und
Fig. 12 schematisch ein Balkendiagramm der Treffergenauigkeit gemäß Fig. 11.

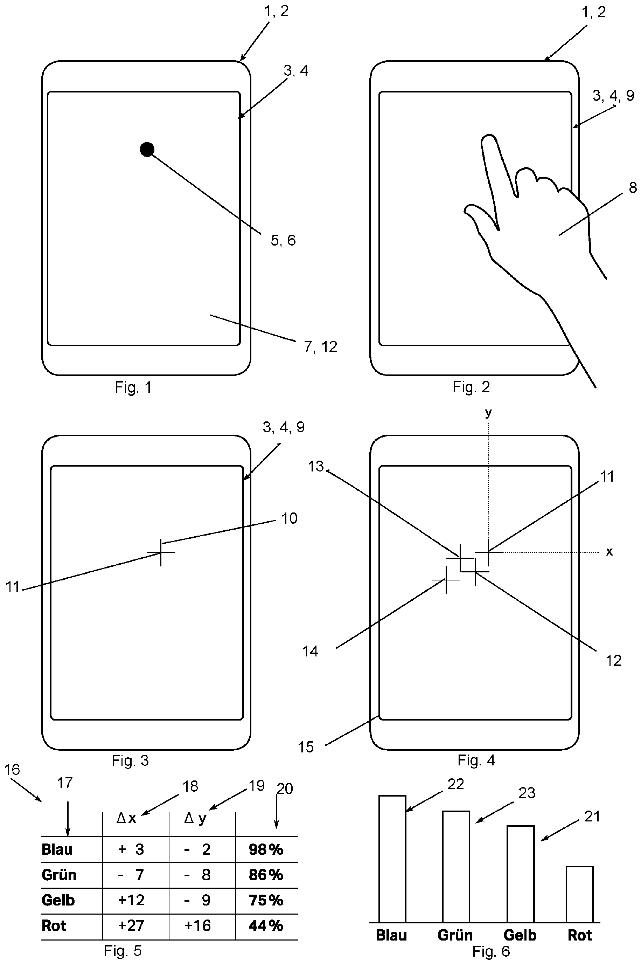

Fig. 1 zeigt eine Vorrichtung 1 zur Datenverarbeitung in Form eines Tablet-PCs 2. Der Tablet-PC 2 weist eine elektronisch gesteuerte Anzeige 3, einen internen Speicher und eine Recheneinheit auf. In dem internen Speicher ist ein Computerprogrammprodukt gespeichert, das Befehle umfasst, die den Tablet-PC 2 zur Ausführung der folgenden Verfahrensschritte veranlasst, wenn es ausgeführt wird. Die Anzeige 3 ist ein integrierter Touchbildschirm 4 des Tablet-PCs 2. Die Anzeige 3 ist zur Anzeige eines Positionshinweises 5, wie dem in diesem Beispiel angezeigten Kreis 6 eingerichtet. Fig. 1 zeigt schematisch den Bildschirminhalt der Anzeige 3 im Rahmen eines computerimplementierten Verfahrens zur optometrischen Farbaustestung, genauer während eines Schrittes zur Messung einer Abweichung einer zeitverzögerten Positionsschätzung eines Nutzers. Dieser Schritt beginnt in diesem Beispiel mit dem Anzeigen eines Positionshinweises 5. Der Positionshinweis 5 entspricht dem Kreis 6 in Fig. 1 und dessen Position innerhalb der Anzeige 3. In der in Fig. 1 gezeigten Situation kann der Kreis 6 beispielsweise in der Farbe Blau gefüllt sein. Die Fläche 7 der Anzeige 3 außerhalb des Kreises 6, d. h. der Hintergrund des Positionshinweises 5, kann beispielsweise in der Farbe Gelb gefüllt sein. Dies würde einer Farbkonfiguration "blau, gelb" entsprechen. In diesem Beispiel wird die gesamte Anzeigefläche der Anzeige 3 als Positionsbereich genutzt, wobei die Erfindung nicht darauf beschränkt ist. Es könnte auch beispielsweise nur ein Teil der Anzeigefläche als Positionsbereich genutzt werden, d. h. innerhalb dessen Positionshinweise angezeigt und Positionseingaben (siehe Fig. 2) akzeptiert werden und/oder der in einer Hintergrundfarbe angezeigt wird.

Nach dem in Fig. 1 gezeigten Schritt wird als nächstes der Positionshinweis 5 in Form des Kreises 6 ausgeblendet. Diese Ausblendung nimmt der Tablet-PC 2 selbstständig nach einer vordefinierten Anzeigedauer von 500 Millisekunden vor. Unmittelbar nach der Ausblendung gibt der Tablet-PC 2 ein Bereitschaftssignal aus, z.B. einen kurzen Signalton ("Beep"). Danach wartet der Tablet-PC 2 einen vordefinierten Zeitabstand von weiteren 500 Millisekunden ab, bevor er mit der Erfassung einer Positionseingabe beginnt.

Der Schritt der Positionseingabe ist schematisch in Fig. 2 gezeigt. Dabei nimmt ein Nutzer des Tablet-PCs 2 die Positionseingabe vor, indem er mit einem Finger seiner Hand 8 die Anzeige 3 des Tablet-PCs 2 berührt. Die Anzeige 3, d.h. der Touchbildschirm 4, fungiert dabei als Positionseingabegerät 9 und erfasst die Stelle oder Position, an der eine Berührung registriert wird. Zu diesem Zeitpunkt ist der Kreis 6 schon mindestens für den vordefinierten Zeitabstand ausgeblendet. Der Nutzer kann daher die Position, an der er den Touchbildschirm 4 berührt, nur auf Basis seiner Erinnerung an den Positionshinweis 5 wählen und seinen Unterarm, seine Hand 8 und seinen Finger entsprechend zu der gewählten Position führen.

Fig. 3 zeigt schematisch ein Fadenkreuz 10, dessen Mittelpunkt der erfassten Eingabeposition 11 entspricht. Das Fadenkreuz 10 kann optional für den Nutzer erkennbar von der Anzeige 3 dargestellt werden, zur Bestätigung der erfassten Eingabeposition 11. In dem hier beschriebenen Ausführungsbeispiel wird es nicht angezeigt, sondern ist in Fig. 3 nur zum Verständnis der Eingabeposition 11 über der Anzeige 3 eingezeichnet.

Die oben beschriebenen Schritte werden im Rahmen des ersten Ausführungsbeispiels weitere drei Male wiederholt, für mindestens zwei verschiedene Farbkonfigurationen. D. h. mindestens bei einer Wiederholung ist mindestens der Positionshinweis 5 oder der Hintergrund 12 in einer anderen Farbe als blau bzw. gelb gefärbt. Für die Zwecke dieses Beispiels nehmen wir an, dass der Kreis 6 bei der ersten Wiederholung grün gefärbt und auf gelbem Hintergrund dargestellt ist, bei der zweiten Wiederholung gelb gefärbt und auf rotem Hintergrund dargestellt ist und bei der dritten Wiederholung rot gefärbt und auf gelbem Hintergrund dargestellt ist. Nach den beispielhaft genannten Wiederholungen wurden somit insgesamt vier Eingabepositionen 11-14 erfasst. Diese Eingabepositionen 11-14 sind in Fig. 4 schematisch auf der Anzeige 3 dargestellt. Jede Eingabeposition 11-14 ist durch zwei Koordinaten, "x, y", in Bezug auf einen Koordinatenursprung 15 "0, 0" der Anzeigefläche der Anzeige 3 definiert. Bei der in Fig. 4 gezeigten Sammlung von Eingabepositionen sind alle vier Eingabepositionen 11-14 sowohl in der horizontalen Koordinate x als auch in der vertikalen Koordinate y voneinander verschieden.

Die Tabelle 16 in Fig. 5 zeigt als Reihen die vier verschiedenen Farbkonfigurationen 17 entsprechend den oben beschriebenen Wiederholungen und als Spalten die Abweichung 18 in Form des geometrischen Abstands Δx zwischen der horizontalen Koordinate der jeweils erfassten Eingabepositionen 11-14 und der horizontalen Koordinate des vor deren Eingabe angezeigten Positionshinweises, die Abweichung 19 in Form des geometrischen Abstands Δy zwischen der vertikalen Koordinate der jeweils erfassten Eingabepositionen 11-14 und der vertikalen Koordinate des vor deren Eingabe angezeigten Positionshinweises, sowie als dritte Spalte die aus den beiden Abständen Δx und Δy ermittelte Treffergenauigkeit 20 der zeitverzögerten Positionsschätzung. Die Treffergenauigkeit 20 wird dabei auf einer Skala von 100 % (keine Abweichung) bis 0 % (maximal zu berücksichtigende Abweichung) angegeben. Die Einheiten der Abstände sind in diesem Beispiel in 0,1 Millimetern (mm) angegeben. D. h. für die erste Farbkonfiguration "Blau" (genauer: "blau, gelb") wurde ein horizontaler Abstand von +0,3 mm und ein vertikaler Abstand von -0,2 mm ermittelt und daraus resultierend eine Treffergenauigkeit von 98 %. Bei der hier verwendeten Skala wird ein linearer Zusammenhang und als maximal zu berücksichtigende Abweichung ein geometrischer Abstand von ca. 5 mm angenommen.

Fig. 6 stellt die in Fig. 5 angegebene Treffergenauigkeit 20 schematisch als Balkendiagramm 21 dar. In diesem Beispiel würde die Farbkonfiguration "Blau" als empfohlene Farbkonfiguration 22 ermittelt werden, weil für diese Farbkonfiguration die höchste Treffergenauigkeit 20 ermittelt wurde. Dieses Ergebnis könnte anschließend auf der Anzeige 3 dem Nutzer angezeigt werden. Weiters könnte dem Nutzer eine Anpassung der Anzeigeeinstellungen des Tablet-PCs 2 vorgeschlagen werden, um beispielsweise Texte in Zukunft in der empfohlenen Farbkonfiguration 22 oder einer dieser empfohlenen Farbkonfiguration näherkommenden Farbkonfiguration darzustellen. Optional könnte dem Nutzer auch zumindest eine alternative Farbkonfiguration 23 empfohlen werden, was in diesem Fall die Farbkonfiguration "Grün" wäre.

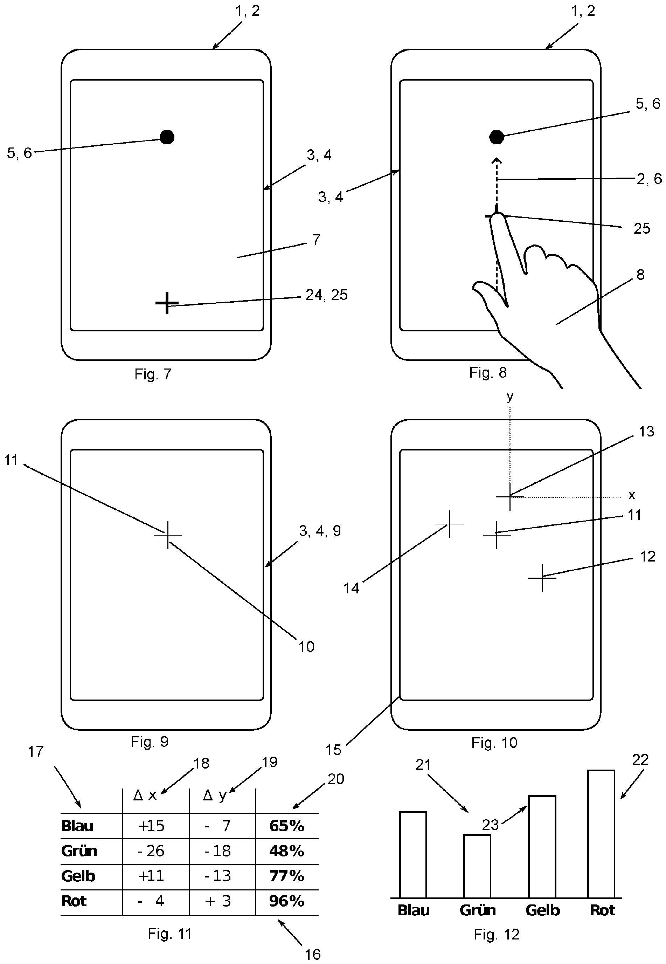

Die Darstellung des zweiten Ausführungsbeispiels des offenbarten Verfahrens in den Figuren 7-12 entspricht in weiten Teilen jener des oben beschriebenen ersten Ausführungsbeispiels. Es werden daher für entsprechende Elemente die gleichen Bezugszeichen verwendet und im jeweiligen Zusammenhang auf die obige Beschreibung verwiesen. Die folgende Beschreibung bezieht sich auf die Unterschiede zwischen den beiden Ausführungsbeispielen.

Anders als in Fig. 1 zeigt die Anzeige 3 bei dem in Fig. 7 dargestellten Schritt außer dem Positionshinweis 5 in Form des Kreises 6 auch eine Eingabemarkierung 24 in Form eines Kreuzes 25 an. Die Eingabemarkierung 24 wird dabei an einer von der Anzeigeposition des Positionshinweises 5 verschiedenen Markierungsposition auf der Anzeige 3 dargestellt. Die Markierungsposition kann vom Tablet-PC 2 zufällig gewählt sein, wobei z.B. nur Markierungspositionen akzeptiert werden, die außerhalb eines vordefinierten Mindestabstands von der Anzeigeposition liegen. Der vordefinierte Mindestabstand kann z.B. 5 cm betragen. Für die Positionseingabe kann der Nutzer wie in Fig. 8 dargestellt mit einem Finger seiner Hand 8 eine Streckeneingabe entlang der als gestrichelter Pfeil 26 dargestellten (jedoch in Anzeige 3 nicht anzeigten) Strecke zwischen der Markierungsposition und der Anzeigeposition vornehmen und gewissermaßen das Kreuz 25 in den Kreis 6 ziehen. Als Eingabeposition wird dabei jene Position erfasst, an der der Nutzer die Bewegung beendet und die Eingabemarkierung 24 "loslässt". Bevor diese Position erreicht wird, wird allerdings der Positionshinweis 5 ausgeblendet, sobald der Benutzer während der Streckeneingabe in einen Umkreis von einem vordefinierten Radius um die Anzeigeposition eintritt. Der vordefinierte Radius kann z.B. 3 cm betragen. Somit ist der Nutzer für den Abschluss der Bewegung auf seine Erinnerung an die Anzeigeposition des Positionshinweises 5 angewiesen und es kann gewährleistet werden, dass tatsächlich eine Positionserinnerung gemessen wird. In diesem Fall kann der Positionshinweis 5 für einen längeren Zeitraum angezeigt werden und muss nicht nach einer vordefinierten Anzeigedauer ausgeblendet werden.

## Patentansprüche

1. Computerimplementiertes Verfahren zur optometrischen Farbaustestung, **gekennzeichnet durch** folgende Schritte:
Messen einer Abweichung (19) einer zeitverzögerten Positionsschätzung eines Nutzers für jede von mindestens zwei verschiedenen Farbkonfigurationen (17),
Ermitteln einer für den Nutzer empfohlenen Farbkonfiguration (22) aus den gemessenen Abweichungen.

2. Computerimplementiertes Verfahren nach Anspruch 1, **dadurch gekennzeichnet, dass** das Ermitteln der für den Nutzer empfohlenen Farbkonfiguration (22) folgende Schritte umfasst:
Vergleichen der gemessenen Abweichungen,
Ermitteln mindestens einer Farbkonfiguration (22), der eine geringere Abweichung (19) zugeordnet ist als mindestens einer anderen Farbkonfiguration (22), als empfohlene Farbkonfiguration (22) .

3. Computerimplementiertes Verfahren nach Anspruch 1 oder 2, **dadurch gekennzeichnet, dass** das Messen der Abweichungen der zeitverzögerten Positionsschätzung je Farbkonfiguration (22) folgende Schritte umfasst:
Anzeigen eines Positionshinweises (5) in der jeweiligen Farbkonfiguration (22) an einer Anzeigeposition auf einer elektronisch gesteuerten Anzeige (3),
Ausblenden des Positionshinweises (5),
Erfassen einer Positionseingabe einer Eingabeposition (11) mit einem Positionseingabegerät nach dem Ausblenden des Positionshinweises (5),
Ermitteln einer Abweichung (19) zwischen der Eingabeposition (11) und der Anzeigeposition als Abweichung (19) der zeitverzögerten Positionsschätzung.

4. Computerimplementiertes Verfahren nach Anspruch 3, **dadurch gekennzeichnet, dass** das Erfassen einer Positionseingabe folgende Schritte umfasst:
Anzeigen einer Eingabemarkierung (24) an einer von der Anzeigeposition verschiedenen Markierungsposition auf der elektronisch gesteuerten Anzeige (3),
Erfassen einer Streckeneingabe einer Verbindungsstrecke von der Markierungsposition bis zu der zu erfassenden Eingabeposition (11) mit dem Positionseingabegerät.

5. Computerimplementiertes Verfahren nach Anspruch 3 oder 4, **gekennzeichnet durch**:
Speichern der erfassten Eingabeposition (11) oder einer ermittelten Abweichung (19) mit einer Zuordnung zu der jeweiligen Farbkonfiguration (22).

6. Computerimplementiertes Verfahren nach einem der Ansprüche 3 bis 5, **dadurch gekennzeichnet, dass** das Erfassen einer Positionseingabe nach einem vordefinierten Zeitabstand nach dem Ausblenden des Positionshinweises (5) beginnt.

7. Computerimplementiertes Verfahren nach einem der Ansprüche 3 bis 6, **gekennzeichnet durch**:
Ausgeben eines Bereitschaftssignals nach dem Ausblenden des Positionshinweises (5).

8. Computerimplementiertes Verfahren nach Anspruch 7, **gekennzeichnet durch**:
Erfassen einer Eingabeverzögerung zwischen dem Ausgeben des Bereitschaftssignals und der Positionseingabe.

9. Computerimplementiertes Verfahren nach Anspruch 8, **gekennzeichnet durch**:
Speichern der erfassten Eingabeverzögerung mit einer Zuordnung zu der jeweiligen Farbkonfiguration (22).

10. Computerimplementiertes Verfahren nach einem der Ansprüche 3 bis 9, **dadurch gekennzeichnet, dass** die Anzeigeposition bei jeder Messung zufällig innerhalb zumindest eines Teils des Anzeigebereichs der elektronisch gesteuerten Anzeige (3) festgelegt wird.

11. Computerimplementiertes Verfahren nach einem der Ansprüche 3 bis 10, **dadurch gekennzeichnet, dass** die Farbkonfiguration (22) aus einer vordefinierten Liste von Farbkonfigurationen (17) durchlaufen werden, wobei die Reihenfolge der Farbkonfigurationen (17) vor jedem Durchlauf zufällig festgelegt wird.

12. Computerimplementiertes Verfahren nach einem der Ansprüche 3 bis 11, **dadurch gekennzeichnet, dass** die vordefinierte Anzeigedauer kürzer als 1 Sekunde, insbesondere kürzer als 500 Millisekunden, vorzugsweise kürzer als 100 Millisekunden ist.

13. Vorrichtung (1) zur Datenverarbeitung, wie Handy, Tablet, Laptop, PC oder AR-System, umfassend Mittel zur Ausführung der Schritte des Verfahrens nach einem der Ansprüche 1 bis 12.

14. Computerprogrammprodukt, umfassend Befehle, die bei der Ausführung des Programms durch die in Anspruch 13 offenbarte Vorrichtung zur Datenverarbeitung diese veranlassen, die Schritte des Verfahrens nach einem der Ansprüche 1 bis 12 auszuführen.

15. Computerlesbares Speichermedium, umfassend Befehle, die bei der Ausführung durch die in Anspruch 13 offenbarte Vorrichtung zur Datenverarbeitung diese veranlassen, die Schritte des Verfahrens nach einem der Ansprüche 1 bis 12 auszuführen.

## Claims

1. Computer-implemented method for optometric colour testing, **characterised by** the following steps:
Measuring a deviation (19) of a time-delayed position estimate of a user for each of at least two different colour configurations (17),
Determining a colour configuration (22) recommended for the user from the measured deviations.

2. Computer-implemented method according to claim 1, **characterised in that** determining the colour configuration recommended for the user (22) comprises the following steps:
Comparing the measured deviations,
Determining at least one colour configuration (22), which is associated with a smaller deviation (19) than at least one other colour configuration (22), as a recommended colour configuration (22).

3. Computer-implemented method according to either claim 1 or claim 2, **characterised in that** measuring the deviations of the time-delayed position estimate per colour configuration (22) comprises the following steps:
Displaying a position indication (5) in the relevant colour configuration (22) at a display position on an electronically controlled display (3),
Hiding the position indication (5),
Detecting a position input of an input position (11) with a position input device after the position indication (5) has been hidden,
Determining a deviation (19) between the input position (11) and the display position as a deviation (19) of the time-delayed position estimate.

4. Computer-implemented method according to claim 3, **characterised in that** detecting a position input comprises the following steps:
Displaying an input marking (24) at a marking position different from the display position on the electronically controlled display (3),
Detecting a route input of a connecting route from the marking position to the input position (11) to be detected with the position input device.

5. Computer-implemented method according to either claim 3 or claim 4, **characterised by:**
Storing the detected input position (11) or a determined deviation (19) with an assignment to the relevant colour configuration (22).

6. Computer-implemented method according to any of claims 3 to 5, **characterised in that** the detection of a position input begins after a predefined time interval after the position indication (5) has been hidden.

7. Computer-implemented method according to any of claims 3 to 6, **characterised by:**
Outputting a ready signal after the position indication (5) has been hidden.

8. Computer-implemented method according to claim 7, **characterised by:**
Detecting an input delay between the ready signal and the position input being output.

9. Computer-implemented method according to claim 8, **characterised by:**
Saving the detected input delay with an assignment to the relevant colour configuration (22).

10. Computer-implemented method according to any of claims 3 to 9, **characterised in that** the display position is randomly determined for each measurement within at least part of the display region of the electronically controlled display (3).

11. Computer-implemented method according to any of claims 3 to 10, **characterised in that** the colour configuration (22) is run through from a predefined list of colour configurations (17), the order of the colour configurations (17) being randomly determined before each run-through.

12. Computer-implemented method according to any of claims 3 to 11, **characterised in that** the predefined display duration is shorter than 1 second, in particular shorter than 500 milliseconds, preferably shorter than 100 milliseconds.

13. Device (1) for data processing, such as a mobile phone, tablet, laptop, PC or AR system, comprising means for carrying out the steps of the method according to any of claims 1 to 12.

14. Computer program product comprising commands which, when the program is executed by the data processing device disclosed in claim 13, cause the device to carry out the steps of the method according to any of claims 1 to 12.

15. Computer-readable storage medium comprising commands which, when executed by the data processing device disclosed in claim 13, cause the device to carry out the steps of the method according to any of claims 1 to 12.

## Revendications

1. Procédé implémenté par ordinateur pour des tests de couleurs optométriques, **caractérisé par** les étapes suivantes :
mesure d'un écart (19) d'une évaluation de position à retardement d'un utilisateur pour chacune d'au moins deux configurations de couleurs (17) différentes,
détermination d'une configuration de couleurs (22) recommandée pour l'utilisateur à partir des écarts mesurés.

2. Procédé implémenté par ordinateur selon la revendication 1, **caractérisé en ce que** la détermination de la configuration de couleurs (22) recommandée pour l'utilisateur comprend les étapes suivantes :
comparaison des écarts mesurés,
détermination d'au moins une configuration de couleurs (22) à laquelle correspond un écart (19) inférieur à celui d'au moins une configuration de couleurs (22) différente de la configuration de couleurs (22) recommandée.

3. Procédé implémenté par ordinateur selon la revendication 1 ou 2, **caractérisé en ce que** la mesure des écarts de l'évaluation de position à retardement comprend, pour chaque configuration de couleur (22), les étapes suivantes :
affichage d'une indication de position (5) dans la configuration de couleur (22) respective au niveau d'une position d'affichage sur un affichage contrôlé électroniquement (3),
masquage de l'indication de position (5),
saisie d'une entrée de position d'une position d'entrée (11) avec un appareil d'entrée de position après le masquage de l'indication de position (5),
détermination d'un écart (19) entre la position d'entrée (11) et la position d'affichage en tant qu'écart (19) de l'évaluation de position à retardement.

4. Procédé implémenté par ordinateur selon la revendication 3, **caractérisé en ce que** la saisie d'une entrée de position comprend les étapes suivantes :
affichage d'un marquage d'entrée (24) au niveau d'une position de marquage différente de la position d'affichage, sur l'affichage contrôlé électroniquement (3).
saisie d'une entrée de trajet d'un trajet de liaison de la position de marquage jusqu'à la position d'entrée (11) à saisir avec l'appareil d'entrée de position.

5. Procédé implémenté par ordinateur selon la revendication 3 ou 4, **caractérisé par** :
l'enregistrement de la position d'entrée (11) saisie ou d'un écart (19) déterminé avec une mise en correspondance avec la configuration de couleurs (22) respective.

6. Procédé implémenté par ordinateur selon l'une des revendications 3 à 5, **caractérisé en ce que** la saisie d'une entrée de position commence après un intervalle de temps prédéfini après le masquage de l'indication de position (5).

7. Procédé implémenté par ordinateur selon l'une des revendications 3 à 6, **caractérisé par** :
l'émission d'un signal d'attente après le masquage de l'indication de position (5).

8. Procédé implémenté par ordinateur selon la revendication 7, **caractérisé par** :
la saisie d'un retardement d'entrée entre l'émission du signal d'attente et l'entrée de position.

9. Procédé implémenté par ordinateur selon la revendication 8, **caractérisé par** :
l'enregistrement du retardement d'entrée avec une mise en correspondance avec la configuration de couleurs (22) respective.

10. Procédé implémenté par ordinateur selon l'une des revendications 3 à 9, **caractérisé en ce que** la position d'affichage est définie, à chaque mesure, de manière aléatoire, dans au moins une partie de la zone d'affichage de l'affichage contrôlé électroniquement (3).

11. Procédé implémenté par ordinateur selon l'une des revendications 3 à 10, **caractérisé en ce que** la configuration de couleurs (22) est déterminée à partir d'une liste prédéfinie de configurations de couleurs (17), dans lequel l'ordre des configurations de couleurs (17) est défini de manière aléatoire avant chaque itération.

12. Procédé implémenté par ordinateur selon l'une des revendications 3 à 11, **caractérisé en ce que** la durée d'affichage prédéfinie est inférieure à 1 seconde, plus particulièrement inférieure à 500 millisecondes, de préférence inférieure à 100 millisecondes.

13. Dispositif (1) pour le traitement de données, comme un téléphone portable, une tablette, un ordinateur portable, un PC ou un système AR, comprenant des moyens pour l'exécution des étapes du procédé selon l'une des revendications 1 à 12.

14. Produit de programme informatique comprenant des instructions qui, lors de l'exécution du programme par le dispositif pour le traitement de données décrit dans la revendication 13, font en sorte que celui-ci exécute les étapes du procédé selon l'une des revendications 1 à 12.

15. Support d'enregistrement lisible par un ordinateur, comprenant des instructions qui, lors de l'exécution par le dispositif pour le traitement de données décrit dans la revendication 13, font en sorte que celui-ci exécute les étapes du procédé selon l'une des revendications 1 à 12.
